# EUROPEAN PATENT SPECIFICATION

(11) **EP 3 228 346 B1**
(45) Date of publication and mention of the grant of the patent: **23.01.2019**
(21) Application number: 17161452.2
(22) Date of filing: 16.03.2017
(51) Int. Cl.: A61M 15/00, A61M 11/00, B05B 17/06

(54) **PORTABLE NEBULIZER WITH A DUST SHIELD**
TRAGBARER ZERSTÄUBER MIT EINEM STAUBSCHUTZ
NÉBULISEUR PORTATIF MUNI D'UN PARE-POUSSIÈRE

(30) Priority: 08.04.2016 CN 201610216563
(43) Date of publication of application: 11.10.2017
(73) Proprietor: Lee, Wen-Ching, Taichung City 408 (TW)
(72) Inventor: Lee, Wen-Ching, Taichung City 408 (TW)
(74) Representative: Becker Kurig Straus

(56) References cited:
- EP-A1- 2 143 457
- WO-A1-2012/009706
- WO-A2-2012/041938
- US-A- 4 809 914
- US-A1- 2007 221 218

## Description

### BACKGROUND OF THE INVENTION

### 1. Field of the Invention

The present invention relates to nebulizer technology and more particularly, to a portable nebulizer that is equipped with a dust shield.

### 2. Description of the Related Art

A nebulizer is a device that turns liquid into a mist of fine droplets. It has a wide range of applications, for example, it can be used for real life application to increase the humidity and to make changes in environmental changes, or for medical application to turn medicine liquid into a mist.

Because a residual mist will be left around the nozzle hole of the nebulizer after each use of the nebulizer, causing dust to be adhered thereto, thus, after a long use, the nozzle hole and atomizer of the nebulizer tends to be covered with dust, affecting the atomization performance of the atomizer.
Portable nebulizers comprising slidable covers for protecting nozzles from dust are known for example from documents EP2143457A1, WO2012/009706A1, US2007/221218A1 or WO2012/041938A2. EP2143457A1 discloses a device having features as defined in the preamble of the appended claim 1.

### SUMMARY OF THE INVENTION

The invention is defined by the appended claims.

The present invention has been accomplished under the circumstances in view.

It is therefore the main object of the present invention to provide a portable nebulizer, which is equipped with a dust shield that is capable of protecting the nozzle hole and atomizer plate of the nebulizer body of the portable nebulizer against environmental contamination.

To achieve this and other objects of the present invention, a portable nebulizer comprises a nebulizer body and a dust shield. The nebulizer body comprises a nozzle hole, and an atomizer plate positioned in the nozzle hole. The dust shield is coupled to the nebulizer body, and movable relative to the nebulizer body between a dust protection position where the dust shield shields the nozzle hole of the nebulizer body and an open position where the dust shield is kept away from the nozzle hole of the nebulizer body.

Thus, the dust shield is capable of protecting the nozzle hole of the portable nebulizer against environmental contamination.

Other advantages and features of the present invention will be fully understood by reference to the following specification in conjunction with the accompanying drawings, in which like reference signs denote like components of structure.

### BRIEF DESCRIPTION OF THE DRAWINGS

FIG. 1 is an elevational view of a portable nebulizer with a dust shield thereof in an open position in accordance with a first embodiment of the present invention.
FIG. 2 corresponds to FIG. 1, illustrating the dust shield in a dust protection position.
FIG. 3 is an exploded view of the portable nebulizer in accordance with the first embodiment of the present invention.
FIG. 4 is a sectional view of the portable nebulizer in accordance with the first embodiment of the present invention.
FIG. 5 is a sectional view of the dust shield of the portable nebulizer in accordance with the first embodiment of the present invention.
FIG. 6 is a sectional view of the first embodiment of the present invention, illustrating the structure of the alternate form of the dust shield of the portable nebulizer.
FIG. 7 is a schematic drawing illustrating a dust shield of a portable nebulizer in an open position in accordance with an alternative solution not belonging to the present invention.
FIG. 8 corresponds to FIG. 7, illustrating the dust shield in a dust protection position.

### DETAILED DESCRIPTION OF THE INVENTION

The composition, effects and features of the present invention will be more fully understood by way of examples in conjunction with the accompanying drawings, in which the components, size and outer appearance of the portable nebulizer are simply for explanation of the technical features of the present invention but not intended for use as limitations.

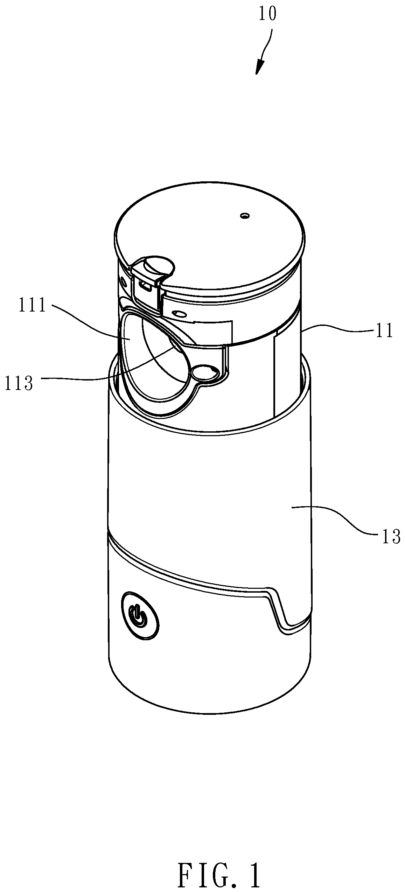

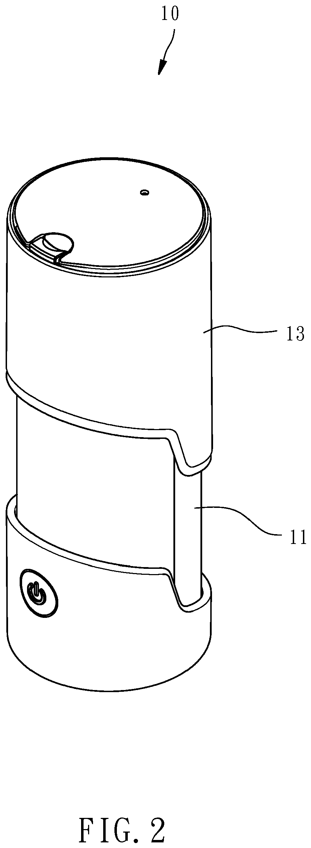

Referring to FIGS. 1 and 2, a portable nebulizer **10** in accordance with a first embodiment of the present invention is shown. The portable nebulizer **10** comprises a nebulizer body **11** and a dust shield **13.** The nebulizer body **11** comprises a nozzle hole **111**, and an atomizer plate **113** disposed inside the nozzle hole **111**. The dust shield **13** is coupled to the nebulizer body **11** and movable relative to the nebulizer body **11** between an open position (see FIG. 1) and a dust protection position (see FIG. 2).

When the dust shield **13** is located at the open position, the dust shield **13** is kept away from the nozzle hole **111** of the nebulizer body **11**, enabling the mist that is transformed from a liquid by the atomizer plate **113** to be spayed out of the nozzle hole **111** of the nebulizer body **11**. When the dust shield **13** is located at the dust protection position, the dust shield **13** shields the nozzle hole **111** of the nebulizer body **11**, preventing adherence of surrounding cotton dust and dirt to the nozzle hole **111** and atomizer plate **113** of the portable nebulizer **10**.

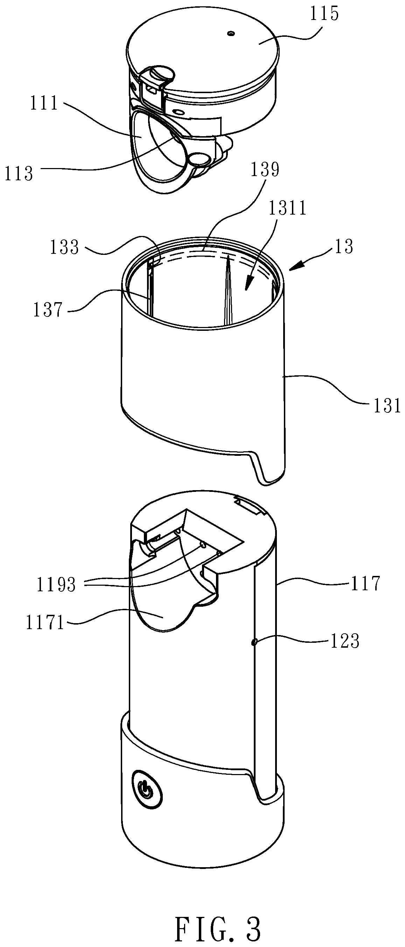

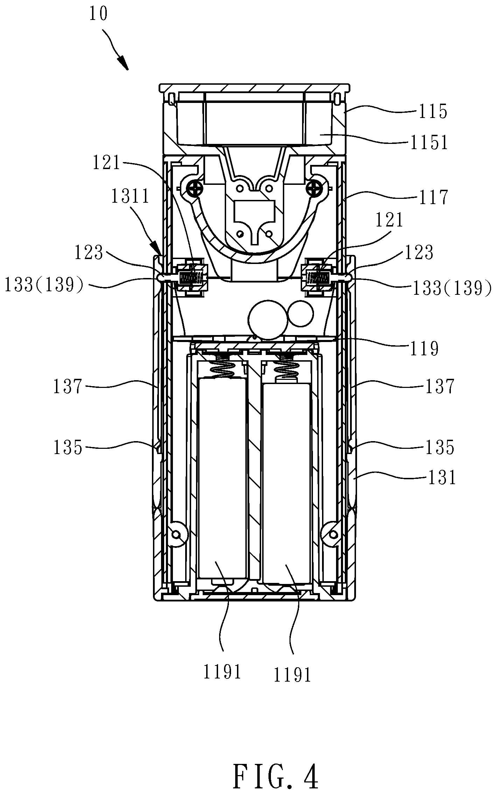

As illustrated in FIG. 3 and FIG. 4, the nebulizer body **11** further comprises a first body shell **115**, a second body shell **117** and an electronic driver **119**. The first body shell **115** defines therein a liquid chamber **1151** adapted for holding water, essential oil, liquid medicine, etc. The atomizer plate **113** is connected to the first body shell **115**, and disposed inside the liquid chamber **1151**. The nozzle hole **111** is located on the outer surface of the first body shell **115.** The electronic driver **119** is fixedly connected to the second body shell **117** and disposed inside the second body shell **117**. In this first embodiment, the electronic driver **119** comprises a driving circuit (not shown), and two battery cells **1191** for providing the driving circuit with the necessary working electricity. The driving circuit **119** can be selected from a circuit composition known in the art, and therefore, we do not repeat it here. The battery cells **1191** can be secondary battery cells, or replaceable battery cells. The first body shell **115** is detachably mounted on the second body shell **117.** After mounted the first body shell **115** on the second body shell **117**, the atomizer plate **113** in the first body shell **115** is electrically connected to the electronic driver **119** in the second body shell **117** so that the electronic driver **119** is controllable to drive the atomizer plate **113** in generating a mist.

As illustrated in FIG. 3, the second body shell **117** defines therein an accommodation chamber **1171** adapted for accommodating a lower half of the first body shell **115** to let the electrodes (not shown) of the atomizer plate **113** of the first body shell **115** be electrically connected to the respective electrodes **1193** of the electronic driver **119** in the accommodation chamber **1171** of the second body shell **117**. The structure of the electrodes of the atomizer plate **113** of the first body shell **115** and the electrodes **1193** of the electronic driver **119** and the connection therebetween are well known in the art, and therefore, we do not repeat them.

The nebulizer body **11** further comprises two springs **121** and two protruding rods **123.** The two springs **121** are respectively mounted inside the second body shell **117.** The two protruding rods **123** are respectively connected to the springs **121** and protruded over the outer surface of the second body shell **117.** When the protruding rods **123** are forced to retract by an external force, the springs **121** are compressed by the respective protruding rods **123.** When the external force disappears, the springs **121** immediately push the respective protruding rods **123** out of the outer surface of the second body shell **117.** In this first embodiment, the springs **121** and the protruding rods **123** can be configured and assembled to create pogo pins, however, the use of pogo pins is not a limitation.

The dust shield **13** in this embodiment is a sliding sleeve **131**. The sliding sleeve **131** defines therein a hollow passage **1311**, two open recesses **133** and two dust protection recesses **135**. The two dust protection recesses **135** and the two open recesses **133** are respectively located on the sliding sleeve **131** and disposed in communication with the hollow passage **1311**. The second body shell **117** is disposed inside the hollow passage **1311** of the sliding sleeve **131**. When the two protruding rods **123** are respectively engaged into the two open recesses **133**, the dust shield **13** is held in the open position. When the two protruding rods **123** are respectively engaged into the dust protection recesses **135**, the dust shield **13** is held in the dust protection position.

In this first embodiment, when the two protruding rods **123** of the second body shell **117** are kept away from the two dust protection recesses **135** and the two open recesses **133**, the dust shield **13** is freely rotatable. Further, the dust protection recesses **135** and the open recesses **133** are curved inwardly from the inner perimeter of the sliding sleeve **131** toward the outer perimeter of the sliding sleeve **131**. Alternatively, the dust protection recesses **135** and the open recesses **133** can be configured to cut through the inner and outer perimeters of the sliding sleeve **131**. Therefore, the dust protection recesses **135** and the open recesses **133** can be formed on the sliding sleeve **131** in any of various different configurations, further, the number of the dust protection recesses **135** and the open recesses **133** is not limited to 2.

The sliding sleeve **131** further comprises two guide grooves **137**. These two guide grooves **137** are formed on the sliding sleeve **131** with the respective open sides thereof respectively disposed in communication with the hollow passage **1311** of the sliding sleeve **131**. The dust protection recesses **135** and the open recesses **133** are respectively disposed at respective two opposite ends of the guide grooves **137**, thus, the two protruding rods **123** can be respectively arranged in the guide grooves **137**, and moved along the respective guide grooves **137** between the dust protection position and the open position.

In this first embodiment, the two guide grooves **137** of the sliding sleeve **131** are straight grooves, however in actual application, the straight design is not a limitation. For example, the guide grooves **137** can be configured to exhibit an arched or spiral profile. Because the configuration of the guide grooves **137** is not limited to the design shown in the annexed drawings, the open position and the dust protection position are not limited to the arrangement of locating on the two opposite ends of a straight line. Further, because the dust shield **13** is slidably or rotatably coupled to the second body shell **117**, using the dust shield **13** at the second body shell **117** to achieve dust protection should be included within the scope of the spirit of the present invention. Accordingly, the invention is not to be limited to the description (illustration) of the present first embodiment.

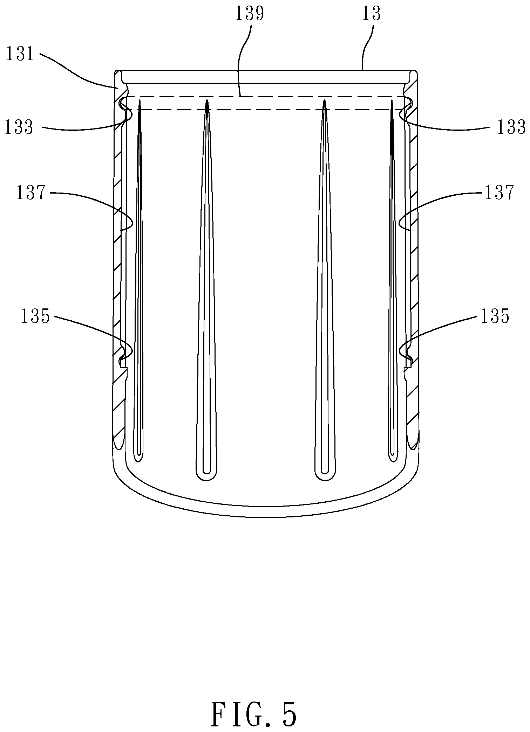

As illustrated in FIG. 5, the dust shield **13** further comprises a conductor **139**. The conductor **139** is connected to the sliding sleeve **131**. The conductor **139** has two opposite ends thereof respectively positioned in the two open recesses **133**. Further, the conductor **139** can be embedded in the sliding sleeve **131**, or connected to the inside wall of the sliding sleeve **131**. The two protruding rods **123** are electrically connected to the electronic driver **119** (see FIG. 4). When the dust shield **13** is located at the open position, the electronic driver **119** is activated. At this time, the two protruding rods **123** are respectively stopped against the two open recesses **133** to achieve electric conduction between the protruding rods **123** and the conductor **139**, therefore, the electronic driver **119** is triggered, driving the atomizer plate **113** to generate a mist. In FIG. 4, the open recesses **133** and the conductor **139** are superimposed, the reference number **139** of the conductor is synchronously indicated in the drawing using a parentheses. When the dust shield **13** is located at dust protection position, the two protruding rods **123** are respectively stopped against the respective dust protection recesses **135**. At this time, the two protruding rods **123** are electrically disconducted to turn off the electronic driver **119**, and therefore, the electronic driver **119** is not activated, and the atomizer plate **113** does no work.

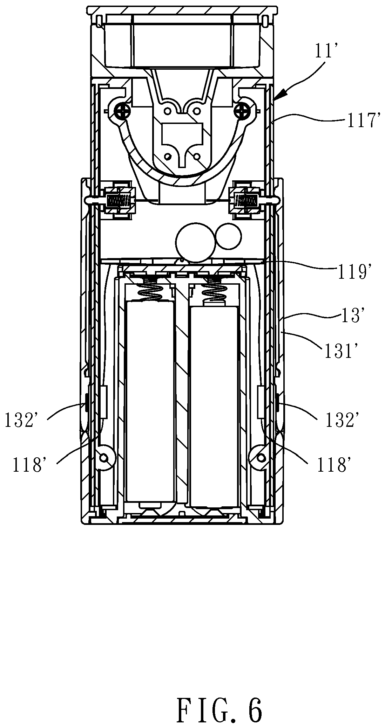

Referring to FIG. 6, an alternate form of the aforesaid dust shield **13** is shown. As illustrated, the dust shield of this alternate form, referenced by **13**', comprises a sliding sleeve **131'** and a magnet **132'**. The magnet **132'** is connected to the sliding sleeve **131'**. Preferably, the magnet **132'** is embedded in the sliding sleeve **131'**. The nebulizer body **11'** further comprises a magnetic sensing element **118'**. The magnetic sensing element **118'** is fixedly connected to the second body shell **117'**, and electrically connected to the electronic driver **119'** for controlling on/off of the electronic driver **119'**. In this embodiment, the magnetic sensing element **118'** is, but not limited to, a Hall sensor. When the sliding sleeve **131'** of the dust shield **13' is** located at the open position, the magnetic sensing element **118'** detected the major part of the magnetic force or the strongest magnetic force of the magnet **132'**, and thus, the magnetic sensing element **118'** triggers the electronic driver **119'**, causing the atomizer plate to work. When the sliding sleeve **131'** of the dust shield **13'** is moved out of the open position or moved to the dust protection position, the magnetic sensing element **118'** is unable to detect the magnetic force of the magnet **132'**, or simply able to detect a micro magnetic force, thus, the electronic driver **119'** is not activated and, the atomizer plate does no work.

Although two methods for triggering the electronic driver **119** are described, however, any other measure can be selectively used to trigger the electronic driver **119** when the dust shield is located at the open position, and therefore, the above-described two electronic driver triggering methods are not intended for use to limit the scope of the present invention.

Further, in the aforesaid first embodiment of the present invention, the sliding sleeve of the dust shield has a hollow passage defined therein, however, in actual application, the sliding sleeve can be configured without the hollow passage, for example, in the form of a half round cover or any other structure capable of covering the nozzle hole, and therefore, the sliding sleeve is not limited to the embodiment described above.

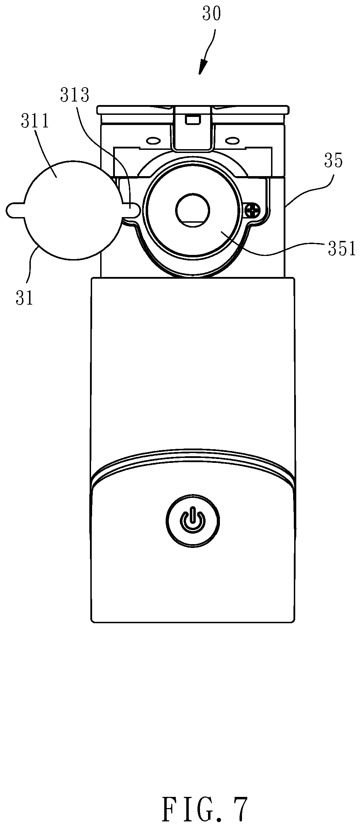

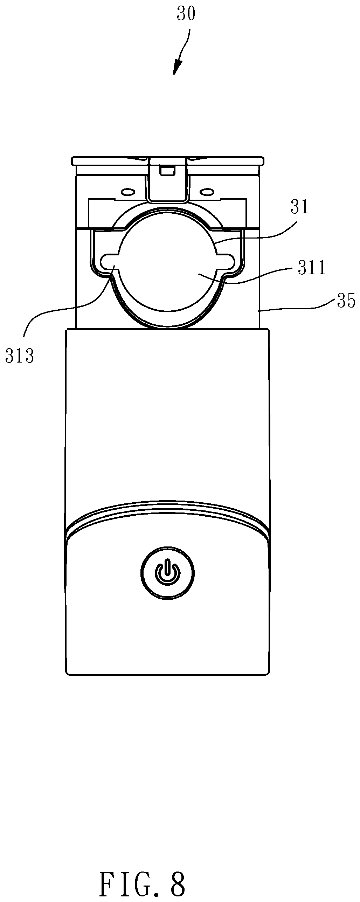

Referring to FIGS. 7 and 8, a portable nebulizer **30** in accordance with an alternative solution is shown. This structure is substantially similar to the aforesaid first embodiment with the exceptions as described hereinafter. The dust shield **31** is not slidably sleeved onto the second body shell **33**. In this alternative, the dust shield **31** comprises a dust protection sheet **311** and a connecting strip **313**. The connecting strip **313** is extended from the border edge of the dust protection sheet **311** and pivotally connected to the first body shell **35** so that the dust shield **31** can be rotated or biased relative to the first body shell **35**.

When the dust shield **31** is located at the dust protection position, the dust protection sheet **311** shields the nozzle hole **351** of the first body shell **35**. Further, the dust protection sheet **311** of the dust shield **31** can be moved away from the dust protection position, enabling the dust shield **31** to be positioned in the open position. In this alternative, the dust protection sheet **311** is biasable and rotatable with the connecting strip **313** relative to the first body shell **35**. The dust shield **31** is preferably made from silicon rubber, rubber, or elastic plastics. Further, the shape of the dust shield is not limited to that described in the specification and illustrated in the drawings.

As described above, the dust shield and the nebulizer body can be connected together in any of various measures, avoid disconnection of the dust shield from the nebulizer body and lost. Further, in the aforesaid two embodiments, the nebulizer body consists of a first body shell and second body shell, however, in actual application, the nebulizer body can be a one piece member, and therefore, the structure of the nebulizer body is not limited to the aforesaid first and second embodiments.

Although particular embodiments of the invention have been described in detail for purposes of illustration, various modifications and enhancements may be made without departing from the scope of the invention. Accordingly, the invention is not to be limited except as by the appended claims.

## Claims

1. A portable nebulizer (10,30), comprising a nebulizer body (11,11') comprising a nozzle hole (111,351) and an atomizer plate (113) positioned in said nozzle hole (111,351), wherein said portable nebulizer (10,30) comprises a dust shield (13,13',31) coupled to said nebulizer body (11,11') and movable relative to said nebulizer body (11,11') between a dust protection position where said dust shield (13,13',31) shields said nozzle hole (111,351) of said nebulizer body (11,11') and an open position where said dust shield (13,13',31) is kept away from said nozzle hole (111,351) of said nebulizer body (11, 11'),
**characterized in that** said nebulizer body (11,11') comprises at least one protruding rod (123); said dust shield (13,13',31) comprises a sliding sleeve (131,131'), said sliding sleeve (131,131') comprises a hollow passage (1311), at least one dust protection recess (135) and at least one open recess (133), said at least one dust protection recess (135) and said at least one open recess (133) being disposed in communication with said hollow passage (1311), said nebulizer body (11,11') being disposed in said hollow passage (1311) of said sliding sleeve (131,131'), said at least one protruding rod (123) of said nebulizer body (11,11') being respectively stopped against said at least one dust protection recess (135) of said sliding sleeve (131,131') when said dust shield (13,13',31) is located at said dust protection position, said at least one protruding rod (123) of said nebulizer body (11,11') being respectively stopped against said at least one open recess (133) of said sliding sleeve (131,131') when said dust shield (13,13',31) is located at said open position, wherein said nebulizer body (11,11') further comprises at least one spring (121) respectively connected with said at least one protruding rod (123) to support said at least one protruding rod (123) in a protruded position protruding over an outer surface of said nebulizer body (11,11').

2. The portable nebulizer (10,30) as claimed in claim 1, wherein said nebulizer body (11,11') further comprises a first body shell (115,35), a second body shell (117,117',33) and an electronic driver (119,119'), said first body shell (115,35) defining therein a liquid chamber (1151); said atomizer plate (113) is connected to said first body shell (115,35) and positioned in said liquid chamber (1151); said nozzle hole (111,351) is located on an outer surface of said first body shell (115,35); said electronic driver (119,119') is connected to said second body shell (117,117',33) and adapted for driving said shell atomizer plate (113) when said first body shell (115,35) is connected to said second body shell (117,117',33); said dust shield (13,13',31) is connected to said second body shell (117,117',33) and movable relative to said second body shell (117,117',33) between said dust protection position and said open position.

3. The portable nebulizer (10,30) as claimed in claim 2, wherein said electronic driver (119,119') is enabled to drive said atomizer plate (113) when said dust shield (13,13',31) is located at said open position; said electronic driver (119,119') is disabled when said dust shield (13,13',31) is located at said dust protection position.

4. The portable nebulizer (10,30) as claimed in claim 3, wherein said at least one protruding rod (123) protruding over an outer surface of said second body shell (117,117',33) and electrically coupled to said electronic driver (119,119'); said dust shield (13,13',31) comprises a conductor (139), said conductor (139) being connected to said sliding sleeve (131,131') and positioned in said at least one open recess (133), said at least one protruding rod (123) being stopped against said conductor (139) in said at least one open recesses (133) to enable said electronic driver (119,119') for triggering said atomizer plate (113) when said dust shield (13,13',31) is located at said open position, said at least one protruding rods (123) being respectively stopped against said at least one dust protection recesses (135) and said electronic driver (119,119') is disabled when said dust shield (13,13',31) is located at said dust protection position.

5. The portable nebulizer (10,30) as claimed in claim 3, wherein said nebulizer body (11,11') further comprises a magnetic sensing element (118') mounted inside said second body shell (117,117',33) and electrically coupled to said electronic driver (119,119'); said dust shield (13,13',31) comprises a magnet (132'), said sliding sleeve (131,131') being connected to said second body shell (117,117',33) and movable relative to said second body shell (117,117',33) between said open position and said dust protection position, said magnet (132') being connected to said sliding sleeve (131,131') and adapted for causing said magnetic sensing element (118') to trigger said electronic driver (119,119') when said sliding sleeve (131,131') is located at said open position, said magnetic sensing element (118') is unable to trigger said electronic driver (119,119') when said sliding sleeve (131,131') is located at said dust protection position

## Patentansprüche

1. Tragbarer Zerstäuber (10,30), welcher umfasst, einen Zerstäuber-Körper (11,11'), umfassend ein Zerstäuber-Düsenloch (111,351) und eine Zerstäuber-Platte (113), die in dem Zerstäuber-Düsenloch (111,351) angeordnet ist, worin der tragbare Zerstäuber (10,30) einen Staubschild (13,13',31) umfasst, der mit dem Zerstäuber-Körper (11,11') gekoppelt und relativ zu dem Zerstäuber-Körper (11,11') zwischen einer Staubschutz-Position, in welcher der Staubschild (13,13',31) das Zerstäuber-Düsenloch (111,351) des Zerstäuber-Körpers (11,11') schützt, und einer Offen-Position, in der der Staubschild (13,13',31) von dem Zerstäuber-Düsenloch (111,351) des Zerstäuber-Körpers (11,11') ferngehalten ist, bewegbar ist,
**dadurch gekennzeichnet, dass** der Zerstäuber-Körper (11,11') mindestens einen vorstehenden Stab (123) umfasst; dass der Staubschild (13,13',31) eine Schiebehülse (131,131') umfasst, worin die Schiebehülse (131,131') umfasst, einen hohlen Durchgang (1311) und mindestens eine Staubschutz-Vertiefung (135) und mindestens eine Offen-Vertiefung (133), worin die mindestens eine Staubschutz-Vertiefung (135) und die mindestens eine Offen-Vertiefung (133) in Verbindung mit dem hohlen Durchgang (1311) angeordnet sind, worin der Zerstäuber-Körper (11,11') in dem hohlen Durchgang (1311) der Schiebehülse (131,131') angeordnet ist, worin der mindestens eine vorstehende Stab (123) des Zerstäuber-Körpers (11,11') jeweils gegen die mindestens eine Staubschutz-Vertiefung (135) der Schiebehülse (131,131') abgestoppt wird, wenn sich der Staubschild (13,13',31) in der Staubschutz-Position befindet, worin der mindestens eine vorstehende Stab (123) des Zerstäuber-Körpers (11,11') jeweils gegen die mindestens eine Offen-Vertiefung (133) der Schiebhülse (131,131') abgestoppt wird, wenn sich der Staubschild (13,13',31) in der Offen-Position befindet, worin der Zerstäuber-Körper (11,11') ferner mindestens eine Feder (121) umfasst, die jeweils mit dem mindestens einen vorstehenden Stab (123) verbunden ist, um den mindestens einen vorstehenden Stab (123) in einer vorstehenden Position über einer äußeren Oberfläche des Zerstäuber-Körpers (11,11') vorstehend zu tragen.

2. Tragbarer Zerstäuber (10,30) nach Anspruch 1, worin der Zerstäuber-Körper (11,11') ferner umfasst, eine erste Körper-Hülle (115,35), eine zweite Körper-Hülle (117,117',33) und eine elektronische Ansteuerung (119,119'), worin die erste Körper-Hülle (115,35) darin eine Flüssigkeitskammer (1151) definiert; worin die Zerstäuber-Platte (113) mit der ersten Körper-Hülle (115,35) verbunden und in der Flüssigkeitskammer (1151) angeordnet ist; worin das Zerstäuber-Düsenloch (111,351) an einer äußeren Oberfläche der ersten Körper-Hülle (115,35) angeordnet ist; worin die elektronische Ansteuerung (119,119') mit der zweiten Körper-Hülle (117,117',33) verbunden und angepasst ist, die Zerstäuber-Platte (113) der Hülle anzutreiben, wenn die erste Körper-Hülle (115,35) mit der zweiten Körper-Hülle (117,117',33) verbunden ist; worin der Staubschild (13,13',31) mit der zweiten Körper-Hülle (117,117',33) verbunden und relativ zu der zweiten Körper-Hülle (117,117',33) zwischen der Staubschutz-Position und der Offen-Position bewegbar ist.

3. Tragbarer Zerstäuber (10,30) nach Anspruch 2, worin die elektronische Ansteuerung (119,119') in der Lage ist, die Zerstäuber-Platte (113) anzutreiben, wenn der Staubschild (13,13',31) in der Offen-Position angeordnet ist; und worin die elektronische Ansteuerung (119,119') deaktiviert ist, wenn der Staubschild (13,13',31) in der Staubschutz-Position angeordnet ist.

4. Tragbarer Zerstäuber (10,30) nach Anspruch 3, worin der mindestens eine vorstehende Stab (123) über eine äußere Oberfläche der zweiten Körper-Hülle (117,117',33) vorsteht und mit der elektronischen Ansteuerung (119,119') elektrisch gekoppelt ist; worin der Staubschild (13,13',31) einen Leiter (139) umfasst, der mit der Schiebehülse (131,131') verbunden und in der mindestens einen Offen-Vertiefung (133) angeordnet ist, worin der mindestens eine vorstehende Stab (123) auf dem Leiter (139) in der mindestens einen Offen-Vertiefung (133) aufliegt, so dass die elektronische Ansteuerung (119,119') die Zerstäuber-Platte (113) aktivieren kann, wenn der Staubschild (13,13',31) in der Offen-Position angeordnet ist, worin der mindestens eine vorstehende Stab (123) jeweils gegen die mindestens eine Staubschutz-Vertiefung (135) abstoppt, und worin die elektronische Ansteuerung (119,119') deaktiviert ist, wenn der Staubschild (13,13',31) in der Staubschutz-Position angeordnet ist.

5. Tragbarer Zerstäuber (10,30) nach Anspruch 3, worin der Zerstäuber-Körper (11,11') ferner ein magnetisches Sensorelement (118') umfasst, das in der zweiten Körper-Hülle (117,117',33) befestigt und mit der elektronischen Ansteuerung (119,119') elektrisch gekoppelt ist; worin der Staubschild (13,13',31) einen Magneten (132') umfasst, worin die Schiebehülse (131,131') mit der zweiten Körper-Hülle (117,117',33) verbunden und relativ zu der zweiten Körper-Hülle (117,117',33) zwischen der Offen-Position und der Staubschutz-Position bewegbar ist, worin der Magnet (132') mit der Schiebehülse (131,131') verbunden und angepasst ist, dass das magnetische Sensorelement (118') die elektronische Ansteuerung (119,119') auslöst, wenn die Schiebehülse (131,131') in der Offen-Position angeordnet ist, und worin das magnetische Sensorelement (118') die elektronische Ansteuerung (119,119') nicht auslösen kann, wenn die Schiebehülse (131,131') in der Staubschutz-Position angeordnet ist.

## Revendications

1. Nébuliseur portatif (10, 30), comprenant un corps de nébuliseur (11, 11') comprenant un trou de buse (111, 351) et une plaque de pulvérisation (113) situés dans ledit trou de buse (111, 351), dans lequel ledit nébuliseur portatif (10, 30) comprend un cache-poussière (13, 13', 31) couplé audit corps de nébuliseur (11, 11') et mobile par rapport audit corps de nébuliseur (11, 11') entre une position de protection contre la poussière dans laquelle ledit cache poussière (13, 13', 31) protège ledit trou de buse (111, 351) dudit corps de nébuliseur (11, 11') et une position ouverte dans laquelle ledit cache de poussière (13, 13', 31) est maintenu à l'écart dudit trou de buse (111, 351) dudit corps de nébuliseur (11, 11'), **caractérisé en ce que** ledit corp de nébuliseur (11, 11') comprend au moins une tige saillante (123) ; ledit cache-poussière (13, 13', 31) comprend un manchon coulissant (131, 131'), ledit manchon coulissant (131, 131') comprend un passage creux (1311), au moins un renfoncement de protection contre la poussière (135) et au moins un renfoncement d'ouverture (133), ledit renfoncement de protection contre la poussière (135) et ledit renfoncement d'ouverture (133) étant agencés en communication avec ledit passage creux (1311), ledit corps de nébulisation (11, 11') étant agencé dans ledit passage creux (1311) dudit manchon coulissant (131, 131'), ladite tige saillante (123) dudit corps de nébuliseur (11, 11') étant respectivement arrêtée contre ledit renfoncement de protection contre la poussière (135) dudit manchon coulissant (131, 131') lorsque ledit cache-poussière (13, 13', 31) est situé dans ladite position de protection, ladite tige saillante (123) du corps de nébuliseur (11, 11') étant respectivement arrêtée contre ledit renfoncement d'ouverture (133) dudit manchon coulissant (131, 131') lorsque ledit cache-poussière (13, 13', 31) est situé dans ladite position ouverte, dans lequel ledit corps de nébuliseur (11, 11') comprend en outre au moins un ressort (121) respectivement relié à ladite tige saillante (123) pour supporter ladite tige saillante (123) dans une position faisant saillie sur une surface extérieure dudit corps de nébuliseur (11, 11').

2. Nébuliseur portatif (10, 30) selon la revendication 1, dans lequel ledit corps de nébuliseur (11, 11') comprend en outre une première enveloppe de corps (115, 35), une deuxième enveloppe de corps (117, 117', 33) et un dispositif de commande électronique (119, 119'), ladite première enveloppe de corps (115, 35) dans laquelle est définie une chambre à liquide (1151), ladite plaque de pulvérisation (113) est reliée à ladite première enveloppe de corps (115, 35) et située dans ladite chambre à liquide (1151) ; ledit trou de buse (111, 351) est situé sur une surface extérieure de ladite première enveloppe de corps (115, 35) ; ledit dispositif de commande électronique (119, 119') est relié à ladite seconde enveloppe de corps (117, 117', 33) et adapté pour entraîner ladite plaque d'atomisation (113) lorsque ladite première enveloppe de corps (115, 35) est reliée à ladite seconde enveloppe de corps (117, 117', 33) ; ledit cache-poussière (13, 13', 31) est relié à ladite seconde enveloppe de corps (117, 177', 33) et est mobile par rapport à ladite seconde enveloppe de corps (117, 117', 33) entre ladite position de protection contre la poussière et ladite position ouverte.

3. Nébuliseur portatif (10, 30) selon la revendication 2, dans lequel ledit dispositif de commande électronique (119, 119') est activé pour entraîner ladite plaque d'atomisation (113) lorsque ledit cache-poussière (13, 13', 31) est situé dans ladite position ouverte ; ledit dispositif de commande électronique (119, 119') est désactivé lorsque ledit cache-poussière (13, 13', 31) est situé dans ladite position de protection contre la poussière.

4. Nébuliseur portatif (10, 30) selon la revendication 3, dans lequel ladite au moins une tige saillante (123) fait saillie sur une surface extérieure de ladite seconde enveloppe de corps (117, 117', 33) et est électriquement couplée audit dispositif de commande électronique (119, 119') ; ledit cache-poussière (13, 13', 31) comprend un conducteur (139), ledit conducteur (139) étant connecté audit manchon coulissant (131, 131') et situé dans ledit renfoncement d'ouverture (133), ladite tige saillante (123) étant bloquée contre ledit conducteur (139) dans ledit renfoncement d'ouverture (133) pour permettre ledit dispositif de commande (119, 119') d'entraîner ladite plaque d'atomisation (113) lorsque ledit cache-poussière (13, 13', 31) est situé dans ladite position ouverte, ladite tige saillante (123) étant respectivement bloquée contre ledit renfoncement de protection contre la poussière (135) et ledit dispositif de commande électronique (119, 119') est désactivé lorsque ledit cache-poussière (13, 13', 31) est situé dans ladite position de protection contre la poussière.

5. Nébuliseur portatif (10, 30) selon la revendication 3, dans lequel ledit corps de nébuliseur (11, 11') comprend en outre un élément de détection magnétique (118') monté à l'intérieur de ladite seconde enveloppe de corps (117, 117', 33) et électriquement couplé audit dispositif de commande électronique (119, 119') ; ledit cache-poussière (13, 13', 31) comprend un aimant (132'), ledit manchon coulissant (131, 131') étant relié à ladite seconde enveloppe de corps (117, 117', 33) et est mobile par rapport à ladite seconde enveloppe de corps (117, 117', 33) entre ladite position ouverte et ladite position de protection contre la poussière, ledit aimant (132') étant relié audit manchon coulissant (131, 131') et adapté pour entraîner ledit élément de détection magnétique (118') pour entraîner ledit dispositif de commande électronique (119, 119') lorsque ledit manchon coulissant (131, 131') est situé dans ladite position ouverte, ledit élément de détection magnétique (118') ne pouvant pas entraîner ledit dispositif de commande électronique (119, 119') lorsque ledit manchon coulissant (131, 131') est situé dans une position de protection contre la poussière.
